# EUROPEAN PATENT APPLICATION

(11) **EP 3 424 350 A1**
(43) Date of publication of application: **09.01.2019**
(21) Application number: 17759941.2
(22) Date of filing: 27.02.2017
(51) Int. Cl.: A23L 33/15, A23L 2/52, A61K 31/4415, A61K 31/519, A61K 31/714, A61P 25/28

(54) **BRAIN HEALTH FOOD OR BRAIN HEALTH BEVERAGE, AND METHOD FOR ADMINISTERING SAME**

(30) Priority: 29.02.2016 JP 2016038284; 17.10.2016 JP 2016203890
(71) Applicant: Park, Kaechang, Kochi-shi Kochi 7808007 (JP)
(72) Inventor: PARK Kaechang, Kochi 7808007 (JP)
(74) Representative: Manuel Illescas y Asociados, S.L.U.
(86) International application number: PCT/JP2017/007573
(87) International publication number: WO 2017/150475

(57) **Abstract**

[Problems to be solved]

To develop a food or beverage for health of brain, for preventing and alleviating brain atrophy due to alcohol intake.

[Means for Solving Problems]

This invention relates to a food or beverage for health of brain containing folic acid, vitamin B6 and vitamin B12 as active ingredients for preventing decrease of brain volume due to drinking of alcohol by habitual drinkers who are not suffering from dementia, i.e., healthy people, wherein the food or beverage for health of brain contains 0.4-0.8 mg of folic acid, 10-20 mg of vitamin B6 and 0.25-0.5 mg of vitamin B12.

## Description

### TECHNICAL FIELD

The present invention relates to food or beverage for health of brain (hereinafter, referred to as "brain health food or beverage"), and method of administering the same, in particular, food or beverage containing folic acid, vitamin B6 and vitamin B12 as ingredients, and method of administering the same.

### BACKGROUND

According to the estimation (2008) of the research group of the Ministry of Health, Labor and Welfare in Japan, the social loss of drinking alcohol causes a loss of 4,148,300 million yen per year. The breakdown of the loss consists of: 1,022,600 million yen in the health care cost for conditions and injuries such as liver disease, cerebral apoplexy and cancer; 3,094,700 million yen in work loss due to conditions or deaths and employment loss such as decrease in productivity; and 28,300 million yen in car crashes, crimes and social security. The social loss caused by the alcohol-related problems, including the problem which is hard to be converted into the amount of money, such as the collapse of the family and an impact on a child, is serious, and in particular, greatly affects health.

It is known that a prolonged constant drinking alcohol would cause various health disorders. Generally, a gastrointestinal cancer such as oral cancer, pharyngeal cancer, laryngeal cancer, esophagus cancer, liver cancer and colon cancer, is well-known, and in particular, a liver-related disease such as a disease causing alcoholic hepatitis or liver cirrhosis and resulting in liver cancer is widely recognized. Researches on indicators to quantitatively monitor liver function have also been advanced and the indicators are widely put into practical use. Therefore, measures for and preventions of health disorders caused by drinking alcohol have been conducted mainly for the liver.

On the other hand, it is drawing attention that habitual drinking alcohol causes damage to brain (brain atrophy).

In 2008, it was reported that brain atrophy may occur even with an alcohol intake which is regarded as an appropriate amount (20 g/day) ("Archives of Neurology" 2008, 65 (10), 1363-1367). For this knowledge, in considering measures for and preventions of health disorders caused by drinking alcohol, it was newly shown that the brain atrophy can be an indicator of brain disorder caused by drinking alcohol as blood concentration of enzyme in liver cells such as GOT and GPT is used as an indicator of liver function disorder. Therefore, it has been desired to monitor changes in brain volume by habitual drinking alcohol, and utilize them in development of food or beverage to prevent and alleviate brain atrophy. However, the mechanism of brain atrophy by alcohol intake is unknown, and thus, such alleviation of brain atrophy was difficult.

The inventor examined the relation between the amount of drinking alcohol and brain atrophy, using a method for measuring brain region volume by brain examination with MRI popular in Japan as a part of health check-ups. As a result, it was confirmed that the total brain volume ratio decreases, i.e., the brain atrophy develops, as the amount of drinking alcohol increases. Then, the inventor started search for factors causing brain atrophy due to alcohol intake by examining the relation between brain atrophy and the blood concentration of various metabolites, using the brain volume value measured with MRI.

Various metabolites are contained in blood, and changes in the concentration thereof involve in the occurrence of any disease and conditions. As one of them, homocysteine is an intermediate metabolite of methionine that is an essential amino acid. It is known that active oxygen is produced in vascular endothelial cells and promotes arteriosclerosis when blood concentration of homocysteine increases. It is also known that homocysteine promotes platelet aggregation by suppressing the production of nitric oxide and finally induces thrombus formation. Furthermore, it has been reported that arteriosclerosis and thrombus formation resulting from such increase of homocysteine cause circulatory disturbance in the brain, thereby leading to cerebral infarction ("Stroke" 2001; 32: 1116-1119).

Folic acid, vitamin B6 and vitamin B12 are known as ingredients which suppress a homocysteine value. Folic acid and vitamin B6 promote metabolism of homocysteine into methionine. Vitamin B12 promotes degradation of homocysteine into cysteine.

Japanese unexamined patent application No. 2009-173640 describes a composition containing vitamin B group consisting of one or more of vitamin B6, vitamin B12 and folic acid, and sesamins.

Japanese unexamined patent application No. 2004-168699 describes a composition containing an extract of lesser periwinkle (extract of Vinca minor) and an extract of ginkgo tree leaves as main ingredients, and containing one or both of docosahexaenoic acid and lecithin, vitamin B6, vitamin B12, and folic acid.

The inventions described in Japanese unexamined patent application No. 2009-173640 and No. 2004-168699 attempt to lower the value of homocysteine in the blood by adding sesamins or docosahexaenoic acid as well as folic acid, vitamin B6 and vitamin B12, and to recover brain function from symptoms of Alzheimer's disease.

Japanese unexamined patent application No. 2009-173640 and No. 2004-168699 report the brain function recovery from Alzheimer's disease, but these have no consideration of the influence on brain atrophy due to drinking of alcohol by healthy people who are not suffering from dementia.

"PloS ONE" 2010, Vol.5, Issue 9, e12244, pp.1-10; " Proceedings of the National Academy of Sciences (PNAS)" 2013, Vol.110, No.23, pp.9523-9528; and "Int.J.Geriatr.Psychiatry" 2012, Vol.27, pp.592-600, also report that folic acid, vitamin B6 and vitamin B12 are administered for preventing brain atrophy caused by MCI (Mild Cognitive Impairment) of elderly people aged 70 years and over, but these ingredients are not administered to the habitual drinkers who are neither suffering from dementia nor MCI, i.e. healthy people.

### SUMMARY OF THE INVENTIION

The inventor focused on the relation between brain atrophy and homocysteine value after monitoring brain volume values measured with MRI for the people drinking a large amount of alcohol habitually, and measuring and analyzing various factors in the blood of such people, in order to seriously search the factors causing brain atrophy due to alcohol intake. Then, the inventor found out that the effect of administration of folic acid, vitamin B6 and vitamin B12 differs depending on genetic polymorphism of methylene tetrahydrofolate reductase (hereinafter referred to as MTHFR) involved in homocysteine metabolism. Moreover, the inventor has attempted to develop food or beverage for preventing and alleviating brain atrophy due to alcohol intake, as if dieting products have been developed by evaluating the diet effects from abdominal girth and BMI. As a result, the inventor had a success in development of food or beverage which can suppress increase of homocysteine, and thus, prevent brain atrophy due to alcohol intake, by containing in the food or beverage the folic acid, vitamin B6 and vitamin B12 having effects of suppressing increase of homocysteine if the blood concentration of homocysteine is 12 mmol/ml or lower and the blood concentration of vitamin B12 is 435-1000 pg/ml in the subject before administration of the invention (Δ condition).

The invention according to first embodiment relates to a brain health food or beverage containing folic acid, vitamin B6 and vitamin B12 as active ingredients for preventing decrease of brain volume due to drinking of alcohol by habitual drinkers who are not suffering from dementia, i.e., healthy people, wherein the brain health food or beverage contains 0.4-0.8 mg of folic acid, 10-20 mg of vitamin B6 and 0.25-0.5 mg of vitamin B12.

The invention according to second embodiment relates to use of the brain health food or beverage containing folic acid, vitamin B6 and vitamin B12 as active ingredients for preventing decrease of brain volume due to drinking of alcohol by habitual drinkers who are not suffering from dementia, i.e., healthy people, wherein the blood concentration of homocysteine is 12 mmol/ml or lower and the blood concentration of vitamin B12 is 435-1000 pg/ml in the healthy people before taking the food or beverage, and wherein the brain health food or beverage contains 0.4-0.8 mg of folic acid, 10-20 mg of vitamin B6 and 0.25-0.5 mg of vitamin B12.

The invention according to third embodiment relates to the use of second embodiment, wherein the above-mentioned healthy people have CC type or CT type as SNP (Single Nucleotide Polymorphism) genetic type of MTHFR.

In embodiments, the term "healthy people" means habitual drinkers (those who have a habit of drinking alcohol every day) who are not suffering from dementia.

### EFFECTS OF THE INVENTION

According to the invention of first embodiment, the homocysteine level in blood of those who take the above-mentioned food or beverage can be decreased by containing 0.4-0.8 mg of folic acid, 10-20 mg of vitamin B6 and 0.25-0.5 mg of vitamin B12 in the food or beverage. This provides an effect that the healthy people (habitual drinkers who are not suffering from dementia) can suppress the decrease of brain volume in the case of alcohol intake for a long term.

According to the invention of second embodiment, the advance determination of the blood concentration of homocysteine or vitamin B12 in healthy people can predict efficacy of brain health food or beverage in the present invention. Thus, the health of habitual drinkers can be promoted more effectively.

According to the invention of third embodiment, the advance determination of the SNP type (CC, CT or TT type) of MTHFR in the healthy people can predict efficacy of brain health food or beverage in the present invention. Thus, the health of habitual drinkers can be promoted more effectively.

### BRIEF DESCRIPTION OF DRAWINGS

Fig. 1 illustrates the relationship between folic acid, vitamin B6 and vitamin B12, and homocysteine metabolism.
Fig. 2 illustrates the comparison of A group (placebo group) with B group (brain health food-taking group) for the blood concentration of homocysteine.
Fig. 3A illustrates the stratification with the decrease of blood concentration of homocysteine and the alleviation reaction of brain atrophy. A group represents placebo group, and B group represents a brain health food-taking group. The group I represents a group in which the homocysteine concentration does not decrease, the group II represents a group in which the homocysteine concentration decreases but the brain atrophy is not alleviated, and the group III represents a group in which the homocysteine concentration decreases and the brain atrophy is alleviated.
Fig. 3B illustrates the frequency of the above-mentioned group I to III in A group (placebo group) and B group (brain health food-taking group).
Fig. 4A illustrates the frequency of the above-mentioned group I to III in A group (placebo group) and B group (brain health food-taking group) with respect to those who have CC type as SNP of MTHFR gene.
Fig. 4B illustrates the frequency of the above-mentioned group I to III in A group (placebo group) and B group (brain health food-taking group) with respect to those who have CT type as SNP of MTHFR gene.
Fig. 4C illustrates the frequency of the above-mentioned group I to III in A group (placebo group) and B group (brain health food-taking group) with respect to those who have TT type as SNP of MTHFR gene.
Fig. 5A illustrates the comparison of the total brain volume ratios in A group (placebo group) and B group (brain health food-taking group) when these groups satisfy a condition (Δ condition) that the blood concentration of homocysteine is 12 mmol/ml or lower and the blood concentration of vitamin B12 is 435-1000 pg/ml in the subjects before receiving the present invention.
Fig. 5B illustrates the comparison of the total brain volume ratios in A group (placebo group) and B group (brain health food-taking group) when these groups do not satisfy the Δ condition.
Fig. 6 illustrates the sites in which the brain volume was increased after taking an agent containing 0.8mg of folic acid, 20mg of vitamin B6 and 0.5mg of vitamin B12 daily for nine months when the subjects (habitual drinkers who are not suffering from dementia, i.e., healthy people) satisfy the
Δ condition.

Fig. 7 illustrates the result of the sense survey for the intake of the present invention if the subjects (habitual drinkers who are not suffering from dementia, i.e., healthy people) satisfy the Δ condition. The subjects received the sense survey after taking an agent containing 0.8mg of folic acid, 20mg of vitamin B6 and 0.5mg of vitamin B12 daily for nine months.

### DETAILED DESCRIPTION OF THE INVENTION

As used herein, the term "healthy people" means habitual drinkers (those who have a habit of drinking alcohol every day) who are not suffering from dementia.

The present invention is a brain health food or beverage containing folic acid, vitamin B6 and vitamin B12 as ingredients for suppressing brain atrophy caused by an increase of homocysteine concentration in a body, wherein the food or beverage may be eaten or taken more than one time per day, wherein the intake per day of the folic acid, vitamin B6 and vitamin B12 may be 0.4-0.8 mg, 10-20 mg, and 0.25-0.5 mg respectively.

The folic acid used for the food or beverage of the invention may be pteroylpolyglutamic acid, pteroylmonoglutamic acid, or a combination thereof.

The vitamin B6 used for the food or beverage may be pyridoxine, pyridoxal, pyridoxamine, their phosphate ester types, i.e., pyridoxine- 5'-phosphoric acid (PNP), pyridoxal- 5'-phosphoric acid (PLP), and pyridoxamine- 5'-phosphoric acid (PMP), or a combination thereof.

The vitamin B12 used for the food or beverage may be hydroxocobalamin, adenosylcobalamin, methylcobalamin, cyanocobalamin, sulfitecobalamin, or a combination thereof.

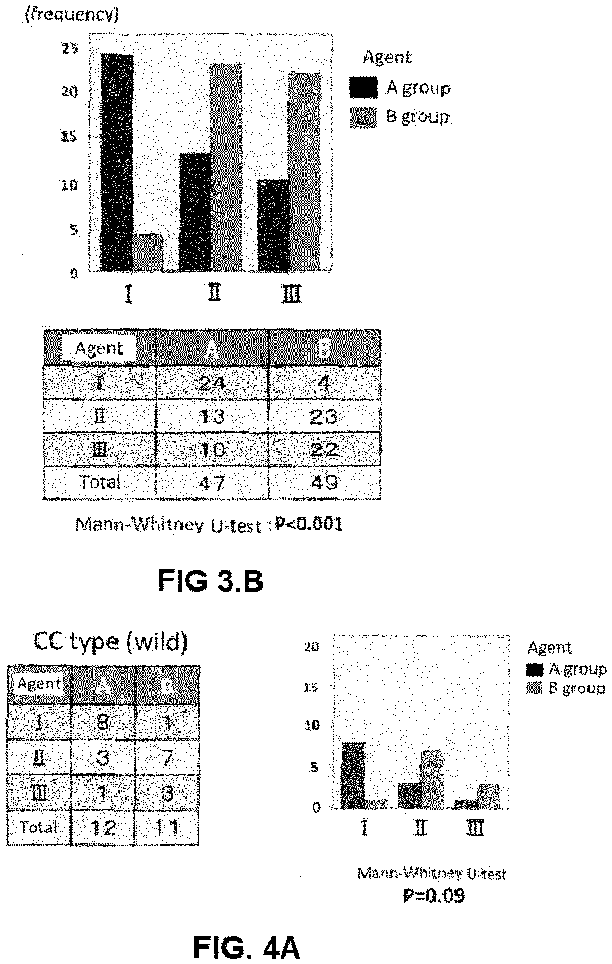

In the case of eating or taking the brain health food or beverage containing 0.4-0.8 mg of folic acid, 10-20 mg of vitamin B6, and 0.25-0.5 mg of vitamin B12 according to the above-mentioned method of eating or taking, a homocysteine value in blood of a human who has taken the above-mentioned food or beverage can be decreased. This can suppress brain atrophy resulting from an alcohol intake for a long-term (see Fig. 3B). The blended amount of these ingredients is 4 to 100 times the amount generally blended in nutritious vitamin preparation, etc. In the invention, the inventor has understood that the effect of the invention can be obtained by blending a large amount of folic acid, vitamin B6 and vitamin B12.

As previously mentioned, the intake per day of folic acid, vitamin B6 and vitamin B12 may be 0.8 mg, 20 mg and 0.5 mg respectively in case of eating or taking the brain health food or beverage of the invention. In CT type of MTHFR, the brain health food or beverage containing folic acid, vitamin B6 and vitamin B12 as ingredients has an effect in suppressing brain atrophy caused by an increase of homocysteine concentration. However, such effect is weak in CC type and not found in TT type (see Fig.4A-C).

For blending into the beverages, since there is a possibility that the ingredients may be decomposed due to the long-term preservation, amounts of 1.8 times the blending amounts of the above-mentioned ingredients are desired. However, in the amounts of 1.8 times, the blending amounts exceed the upper limit value in light of the tolerable upper limit amount under the nutrition label standards. Therefore, actual blending amount may be determined in consideration of these conditions. In CT type of MTHFR, the brain health food or beverage containing folic acid, vitamin B6, and vitamin B12 as ingredients has an effect in suppressing brain atrophy caused by an increase of homocysteine concentration. However, such effect is weak in CC type and not found in TT type (see Fig.4A-C).

### (Control of homocysteine concentration by folic acid, vitamin B6 and vitamin B12)

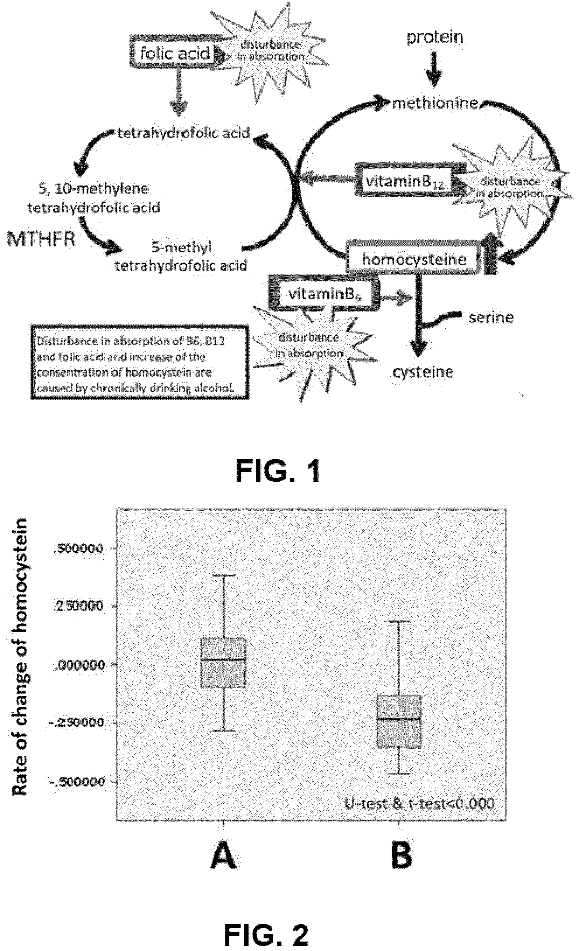

When protein is digested and absorbed, and degraded into an amino acid level, methionine is produced as an essential amino acid. Homocysteine is produced as an intermediate product when the methionine is metabolized in a liver (see Fig. 1).

Folic acid, vitamin B6 and vitamin B12 are essential for the degradation of homocysteine. The folic acid promotes the metabolism of converting homocysteine into methionine via the metabolism of converting 5-methyl tetrahydrofolic acid into tetrahydrofolic acid, wherein the 5-methyl tetrahydrofolic is the metabolite of folic acid. In the pathway, tetrahydrofolic acid is dimethylized, thereby producing 5, 10-methyl tetrahydrofolic acid. 5, 10-methyl tetrahydrofolic acid is further reduced by MTHFR, thereby producing 5-methyl tetrahydrofolic acid.

The vitamin B12 promotes the metabolism of converting homocysteine into methionine. Moreover, the vitamin B6 degrades homocysteine into cysteine.

### (Habitual alcohol intake can increase a homocysteine value via folic acid, vitamin B6 and vitamin B12, and cause reduction of brain volume, i.e., brain atrophy)

Chronic intake of a large amount of alcohol degenerates a gastroenteric mucosal cell and results in a disturbance in absorption of folic acid, vitamin B6 and vitamin B12. Moreover, acetaldehyde resulting from an alcohol intake promotes degradation of folic acid, vitamin B6 and vitamin B12. As a result, the blood concentration of homocysteine increases.

It is known that active oxygen is produced within a vascular endothelial cell when blood concentration of homocysteine increases, and promotes arteriosclerosis. It is also known that homocysteine promotes platelet aggregation by suppressing the production of nitrogen monoxide, and eventually induces thrombosis. Furthermore, it has been reported that the arteriosclerosis and thrombosis cause a circulatory disorder in brain and result in cerebral infarction. Therefore, it can be considered that the increase of the homocysteine concentration resulting from a habitual alcohol intake causes a decreased cerebral circulation and results in brain atrophy.

As mentioned previously, a habitual alcohol intake causes an increase of a homocysteine value, and results in brain atrophy. The inventor has found that an administration of a large amount of folic acid, vitamin B6 and vitamin B12 is effective in decreasing the homocysteine value. A food or beverage containing 0.4-0.8 mg of folic acid, 10-20 mg of vitamin B6 and 0.25-0.5 mg of vitamin B12 can decrease a homocysteine value in blood of a human who has taken the food or beverage (see Fig.2). This can suppress brain atrophy caused by an alcohol intake for a long term and prevent reduction of brain volume.

### EXAMPLES

Although the suitable examples of the invention are described below, the invention is not limited to the contents of the specification and the drawings.

### (Example 1. Evaluation for the effect on brain atrophy by an intake of a brain health food)

Subjects who are not suffering from dementia and habitually drink alcohol (healthy people) were given a brain health food of the invention, and evaluated for the preventive effect on brain atrophy caused by an alcohol intake.

### (Experiment method)

The effect resulting from the intake of the brain food was investigated using a randomized, double-blind trial for 96 healthy volunteers (48 male and 48 female). 30-60 years old (average age: 48.4) healthy people (those who drink alcohol almost every day) were subjected to the experiment, wherein they do not smoke, take no supplements, and had a good state of health including a good liver function.

Subjects were divided into two groups, and the subjects in A group took a placebo and the subjects in B group took an agent containing 0.8 mg of folic acid, 20 mg of vitamin B6, and 0.5 mg of vitamin B12 every day. The subjects were examined for the following examination items on the first day, after three month, and after nine month.
- Measurement of a brain region volume by brain examination with MRI.
- Measurement of blood concentration of vitamin B group, etc.
- Test of gene polymorphism (SNP:MTHFR).

The test was carried out only on the first day.

### (Result)

Firstly, blood concentration of homocysteine was compared between A and B groups. As a result, as shown in Fig. 2, the rate of change in the blood concentration of homocysteine after taking the argent for three month was significantly lowered in B group as compared with A group (A group: 0.0310±0.18467, n=47; B group: -0.2355±0.16925, n=49).

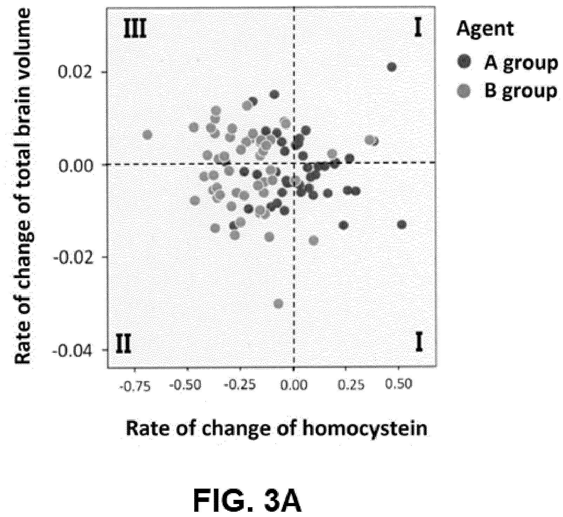

Secondly, an effect on brain atrophy provided by the reduction in blood concentration of homocysteine was evaluated. For the evaluation, the subjects were divided into the following three groups, and then the groups were stratificated based on the reduction of the blood concentrations of homocysteine and the alleviation reaction of brain atrophy (see Fig. 3A).
Group I: a group in which the homocysteine concentration did not decrease.
Group II: a group in which the homocysteine concentration decreased, but the brain atrophy was not alleviated.
Group III: a group in which the blood concentration of homocysteine reduced, and the brain atrophy was alleviated.

Moreover, when the number of people in the three groups I, II and III in A group (placebo group) and B group (brain health food-taking group) were compared and then rank sum test was performed, a significant difference was recognized between A and B groups (Fig. 3B). Accordingly, a proportion of the number of people in group III (the group in which the homocysteine concentration was decreased and the brain atrophy was alleviated) was significantly increased in B group as compared with A group (Mann-Whitney U test: p<0.001).

### (Discussion)

From the above-mentioned result, the blood concentration of homocysteine was decreased by daily intake of 0.8 mg of folic acid, 20 mg of vitamin B6, and 0.5 mg of vitamin B12. Also, brain atrophy was alleviated in relation to the decrease of homocysteine concentration.

### (Example 2. Evaluation of an effect on brain atrophy provided by intake of brain health food, and an effect by MTHFR)

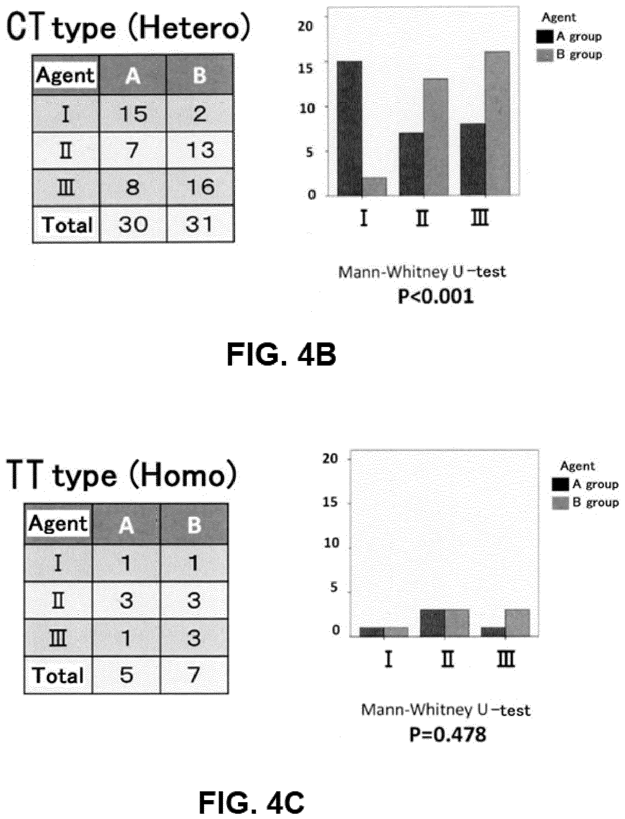

The data obtained in above-mentioned Example 1 was re-examined utilizing SNP genetic types (CC type: wild type, CT type: hetero type, and TT type: homo type) of MTHFR which is a rate-limiting enzyme in homocysteine metabolism (see Fig. 4A-C). Firstly, the subjects were classified into three groups (CC type, CT type, and TT type) based on each three genetic types in SNP of MTHFR. Secondly, the same analysis as Example 1 (the comparison of frequency distribution in group I, II and III between A and B groups) was performed on each subject group. As the result, in CT type (hetero type), a bias of the frequency distribution from group I to III was significant between A and B groups (Mann-Whitney U test: p<0.001) (see Fig. 4B). On the other hand, a tendency of a bias of frequency distribution in CC type (wild type) was similar to that in CT type (hetero type), but the bias of frequency distribution in CC type was not significant between A and B groups (Mann-Whitney U test: p=0.09) (see Fig. 4A). Thus, CT type (hetero type) of MTHFR has an effect in alleviating brain atrophy by intake of brain health food as compared with CC type (wild type). In TT type (homo type), since the activity of MTHFR was low, a significant effect in alleviating the brain atrophy by the intake of brain health food was not recognized (Mann-Whitney U test: p=0.478) (see Fig. 4C).

### (Example 3. Identification of groups in which intake of brain health food alleviates brain atrophy caused by drinking alcohol (Δ condition))

Brain volume measurement by MRI used in above-mentioned Examples 1 and 2 is performed by regularly using VBM (voxel based morphometry) method for measuring each brain region volume of subject's brain by mapping the subject's brain to a standard brain having a pre-determined size, obtaining a probability distribution of brain volume, and then performing the inverse-mapping. However, a more accurate method for measuring is required since an annual variation of healthy people's brain volume is as very small as 1 to 2 ml.

Therefore, in the Example, an analysis was performed by using TBM (tensor based morphometry) method for directly comparing subject's brain volumes between two points of time without using standard brain. In other words, a preventive effect on brain atrophy resulting from alcohol intake was analyzed by comparing all the brain volumes before and after continuously taking the present invention for nine months.

Specifically, the analysis was performed by the following experiment method.

### (Experiment method)

The effect resulting from the intake of the brain food was investigated using a randomized, double-blind trial for 96 healthy volunteers (48 male and 48 female). 30-60 years old (average age: 48.4) healthy people (those who drink alcohol almost every day) were subjected to the experiment, wherein they do not smoke, take no supplements, and had a good state of health including a good liver function.

Subjects were divided into two groups, A and B groups, and a condition of group in which brain atrophy was alleviated in A and B groups was searched nine months after kept administered a placebo for A group and an agent containing 0.8 mg of folic acid, 20 mg of vitamin B6, and 0.5 mg of vitamin B12 for B group every day.

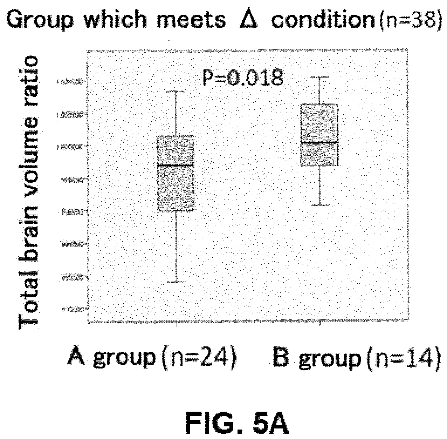

From the search, in the group (38 people: 13 male, 25 female) which meets a condition (Δ condition) in which the blood concentration of homocysteine before administrating the brain health food was 12 mmol/ml or lower and vitamin B12 was limited within the range of 435 to 1000 pg/ml, it was found out that an average value of total brain volume ratio of B group was significantly increased as compared with A group (Fig. 5A: A group 0.99801±0.00310, n=24; B group 1.00041±0.002476, n=14; t-test P=0.018).

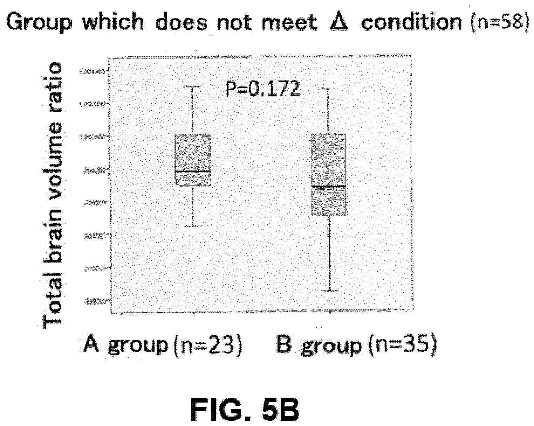

Conversely, there was no significant difference in the group (58 people: 33 male, 25 female) which did not meet the Δ condition (Fig. 5B: A group 0.99823±0.00049, n=23; B group 0.99711±0.00057, n=35; t-test P=0.172).

From this, it was found out that the condition of group in which the brain atrophy was alleviated in habitual alcohol intakes is a condition (Δ condition) in which the blood concentration of homocysteine before administrating the brain health food is 12 mmol/ml or lower and vitamin B12 is limited within the range of 435 to 1000 pg/ml.

### (Example 4: Effect by the Δ condition and MTHFR)

A relation between the data obtained in Example 3 and the SNP genetic type (CC type: wild type, CT type: hetero type, and TT type: homo type) of MTHFR was re-examined. In 38 people who meet the Δ condition, 5 people have CC type, 32 people have CT type, and 1 person has TT type. Thus it can be said that most healthy people who meet the Δ condition have CC type or CT type.

### (Example 5: Brain site in which volume was increased by administrating the present invention)

The subjects who meet the Δ condition were divided into two groups, A and B groups, and subjected to a measurement by TBM method nine months after kept administered a placebo for A group and an agent containing 0.8 mg of folic acid, 20 mg of vitamin B6, and 0.5 mg of vitamin B12 for B group every day.

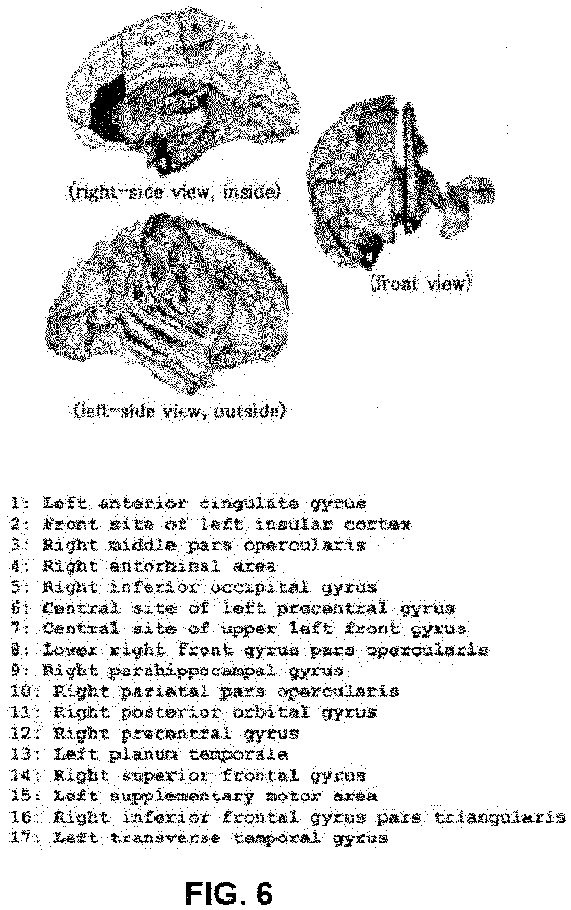

As a result, brain sites where the brain region volume of B group was significantly increased as compared with A group, include the following sites (see Fig. 6).
- 1:: Left anterior cingulate gyrus
- 2:: Front site of left insular cortex
- 3:: Right middle pars opercularis
- 4:: Right entorhinal area
- 5:: Right inferior occipital gyrus
- 6:: Central site of left precentral gyrus
- 7:: Central site of upper left front gyrus
- 8:: Lower right front gyrus pars opercularis
- 9:: Right parahippocampal gyrus
- 10:: Right parietal pars opercularis
- 11:: Right posterior orbital gyrus
- 12:: Right precentral gyrus
- 13:: Left planum temporale
- 14:: Right superior frontal gyrus
- 15:: Left supplementary motor area
- 16:: Right inferior frontal gyrus pars triangularis
- 17:: Left transverse temporal gyrus

From this, the present invention is considered to be effective to increase the volume of the above-mentioned brain sites.

### (Example 6: Sense Survey by Intake of Brain Health Food)

Subjects were divided into two groups, A and B groups, and subjected to a sense survey nine months after kept administered a placebo for A group and an agent containing 0.8 mg of folic acid, 20 mg of vitamin B6, and 0.5 mg of vitamin B12 for B group every day.

Survey questions were as follows:
1. Experienced any change in your physical condition before or after administration of the agent.
2. Drunkenness after drinking an alcohol decreased.
3. Lassitude of the body disappeared or became mild.
4. Became less tired after exercising.
5. Became easy to go to sleep.
6. Became capable of experiencing deep sleep.
7. Began to have an appetite.
8. Constipation reduced.
9. Luster of skin improved.
10. Spread of makeup improved.
11. Became less depressed.

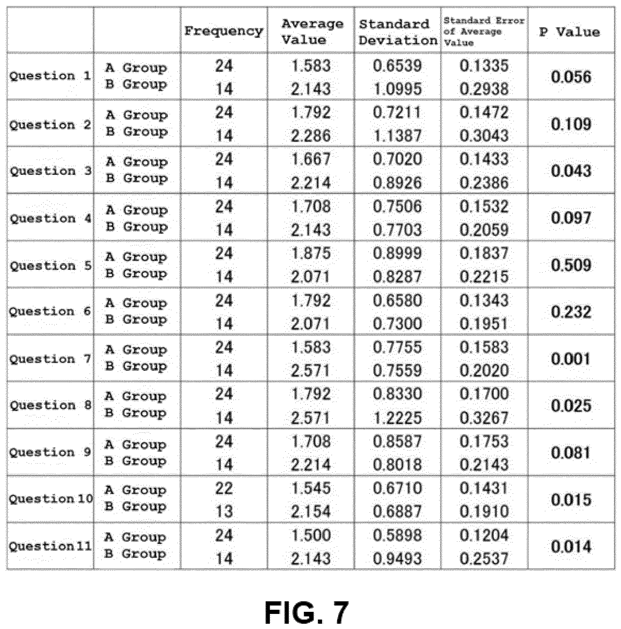

For the above questions, the subjects were evaluated following these scores: 1 - not sensed, 2 - little sensed, 3 - a little sensed, 4 - sensed, and 5 - much sensed. The evaluation results were made into frequency distribution, and from the frequency distribution, average value, standard deviation, and standard error of the average value were calculated. Furthermore, we confirmed whether there is any significant difference between A and B groups in each question by calculating a P value using a t-test. The results are shown in Fig. 7.

As a result of the sense survey, in a group which does not meet the Δ condition, we found out that there is no significant difference between A and B groups in all the questions (not shown).

On the other hand, in a group which does meet the Δ condition, we found out that there is a significant difference with P< 0.05 between A and B groups in the questions 3, 7, 8, 10, and 11, and there is also a significant difference with P< 0.10 between A and B groups in the questions 1 and 4 (see Fig. 7).

From these results, we found out that the subjects who meet the Δ condition and took the brain health food of the present invention significantly showed a positive reaction in the sense survey. On the contrary, the subjects who significantly showed a positive reaction to the above-mentioned sense survey is considered to have a significantly high possibility of meeting the Δ condition.

Examples of the brain health foods according to the present invention include supplements, dietetic foods adapted for medical purposes, edible oils and fats, cream, cheese, powdered milk, meat products, processed seafood products, processed vegetables and fruits, fried tofu pieces [Abura-age], freeze-dried tofu pieces [Kohri-dofu], jelly made from devils' tongue root [Konnyaku], tofu, fermented soybeans, processed eggs, pre-cooked curry stew, stew and soup mixes, dried flakes of laver for sprinkling on rice in hot water [Ochazuke-nori], dried flakes of fish, meat, vegetables or seaweed [Furi-kake], side-dish made of fermented soybean [Name-mono], protein for human consumption, sweet bun, sandwiches, steamed buns stuffed with minced meat [Chuka-manjuh], hamburgers, pizzas, hot dogs, meat pies, seasonings, spices, ice cream mixes, sherbet mixes, cereal preparations, Chinese stuffed dumplings [Gyoza, cooked], Chinese steamed dumplings [Shumai, cooked], sushi, fried balls of batter mix with small pieces of octopus [Takoyaki], ravioli, yeast powder, fermenting malted rice [Koji], yeast, baking powder, instant confectionery mixes, pasta sauce, sake cake, gluten for food, and flour. Preventive effect on the above-mentioned brain atrophy can be achieved by blending 0.4 - 0.8 mg of folic acid, 10 - 20 mg of vitamin B6, and 0.25 - 0.5 mg of vitamin B12, preferably 0.8 mg of folic acid, 20 mg of vitamin B6, and 0.5 mg of vitamin B12, as blending amounts.

Examples of the brain health beverages according to the present invention include dietetic foods adapted for medical purposes, milk, lactic acid drinks, lactic acid bacteria drinks, fermented milk, powdered milk, goat milk, sheep milk, condensed milk and evaporated milk, soya milk, tea, coffee, cocoa, beer, soft drinks, fruit drinks, vegetable juices, whey beverages, sake, western liquors in general, alcoholic fruit beverages, Japanese Shochu-based beverages [Chuhai], Chinese liquors in general, and flavored liquors. Preventive effect on the above-mentioned brain atrophy can be achieved by blending 0.4 - 0.8 mg of folic acid, 10 - 20 mg of vitamin B6, and 0.25 - 0.5 mg of vitamin B12, preferably 0.8 mg of folic acid, 20 mg of vitamin B6, and 0.5 mg of vitamin B12, as blending amounts. Besides, for blending into the beverages, since there is a possibility that the ingredients may be decomposed due to the long-term preservation, amounts of 1.8 times the blending amounts of the above-mentioned ingredients are desired. However, in the amounts of 1.8 times, the blending amounts exceed the upper limit value in light of the tolerable upper limit amount under the nutrition label standards. Therefore, actual blending amount may be determined in consideration of these conditions.

As an intake method for the above-mentioned foods, the above-mentioned 0.4 - 0.8 mg of folic acid, 10 - 20 mg of vitamin B6, and 0.25 - 0.5 mg of vitamin B12, preferably 0.8 mg of folic acid, 20 mg of vitamin B6, and 0.5 mg of vitamin B12, may be taken once a day or twice or more a day.

As an intake method for the above-mentioned beverages, the above-mentioned 0.4 - 0.8 mg of folic acid, 10 - 20 mg of vitamin B6, and 0.25 - 0.5 mg of vitamin B12, preferably 0.8 mg of folic acid, 20 mg of vitamin B6, and 0.5 mg of vitamin B12, may be taken once a day or twice or more a day.

### INDUSTRIAL APPLICABILITY

Since the brain health foods and beverages according to the present invention suppress elevation of a homocysteine level and are effective in preventing a brain atrophy, intake/administration of the brain health foods and beverages leads to health promotion for those who drink alcohol on a daily basis. Moreover, since the brain atrophy-alleviating effect by the brain health foods or beverages varies with SNP of MTHFR, efficacy of the above-mentioned brain health foods and beverages can be predicted by determining a SNP type or a blood concentration of homocysteine or vitamin B12 in advance, allowing for more efficient health promotion for habitual drinkers. Even if a SNP type or a blood concentration cannot be determined in advance, efficacy can be predicted by the sense survey in taking the above-mentioned brain health foods and beverages.

### EXPLANATION OF NUMERALS

- 1:: Left anterior cingulate gyrus
- 2:: Front site of left insular cortex
- 3:: Right middle pars opercularis
- 4:: Right entorhinal area
- 5:: Right inferior occipital gyrus
- 6:: Central site of left precentral gyrus
- 7:: Central site of upper left front gyrus
- 8:: Lower right front gyrus pars opercularis
- 9:: Right parahippocampal gyrus
- 10:: Right parietal pars opercularis
- 11:: Right posterior orbital gyrus
- 12:: Right precentral gyrus
- 13:: Left planum temporale
- 14:: Right superior frontal gyrus
- 15:: Left supplementary motor area
- 16:: Right inferior frontal gyrus pars triangularis
- 17:: Left transverse temporal gyrus

## Claims

1. A food or beverage for health of brain containing folic acid, vitamin B6 and vitamin B12 as active ingredients for preventing decrease of brain volume due to drinking of alcohol by habitual drinkers who are not suffering from dementia, i.e., healthy people, **characterized in that** the food or beverage for health of brain contains 0.4-0.8 mg of folic acid, 10-20 mg of vitamin B6 and 0.25-0.5 mg of vitamin B12.

2. Use of the food or beverage for health of brain containing folic acid, vitamin B6 and vitamin B12 as active ingredients for preventing decrease of brain volume due to drinking of alcohol by habitual drinkers who are not suffering from dementia, i.e., healthy people, **characterized in that** the blood concentration of homocysteine is 12 mmol/ml or lower and the blood concentration of vitamin B12 is 435-1000 pg/ml in the healthy people before taking the food or beverage, and
wherein the food or beverage for health of brain contains 0.4-0.8 mg of folic acid, 10-20 mg of vitamin B6 and 0.25-0.5 mg of vitamin B12.

3. The use of claim 2, **characterized in that** the healthy people have CC type or CT type as SNP genetic type of MTHFR.
